# EUROPEAN PATENT SPECIFICATION

(11) **EP 0 633 893 B1**
(45) Date of publication and mention of the grant of the patent: **24.11.1999**
(21) Application number: 93906670.0
(22) Date of filing: 15.03.1993
(51) Int. Cl.: C07J 43/00, A61K 31/58

(54) **17-SUBSTITUTED STEROIDS USEFUL IN CANCER TREATMENT**
17-SUBSTITUIERTE STEROIDE, VERWENDBAR BEI BEHANDLUNG VON KREBS
STERO DES SUBSTITUES-17 UTILES POUR LE TRAITEMENT DU CANCER

(30) Priority: 31.03.1992 GB 9207057; 27.11.1992 GB 9224880
(43) Date of publication of application: 18.01.1995
(73) Proprietor: BTG INTERNATIONAL LIMITED, London EC4M 7SB (GB)
(72) Inventor: BARRIE, Susan Elaine, Westerham, Kent, TN16 1SJ (GB); JARMAN, Michael, London SW17 7DY (GB); POTTER, Gerard Andrew, Sutton, Surrey SM2 6DW (GB)
(74) Representative: Percy, Richard Keith
(86) International application number: GB9300531
(87) International publication number: WO9320097

(56) References cited:
- EP-A- 0 288 053
- EP-A- 0 413 270
- BULLETIN OF THE POLISH ACADEMY OF SCIENCES. CHEMISTRY vol. 33, no. 1-2, 1985, WARSAW PL pages 19 - 27 J. WICHA ET AL 'Cardiotonic Steroids. Part 8. Synthesis of 17.beta.(3`-pyridyl)-14.be ta.-Androst-4-ene-3.beta.14-diol from 17-Oxoandrostane Derivatives'
- BULLETIN OF THE POLISH ACADEMY OF SCIENCES. CHEMISTRY vol. 32, no. 1-2, 1984, WARSAW PL pages 75 - 83 J. WICHA ET AL 'Cardiotonic Steroids. Part 7. Synthesis of 17.beta.-Pyridyl-androstane Derivatives'
- HETEROCYCLES vol. 16, no. 4, 1981, AMSTERDAM NL pages 521 - 524 J. WICHA ET AL 'Synthesis of 17.beta.-Pyridyl- and 17.beta.-Pyridonyl-androstane Derivatives'
- HETEROCYCLES vol. 20, no. 2, 1983, AMSTERDAM NL pages 231 - 234 J. WICHA ET AL 'Synthesis and Molecular Biological Activity of the Pyridine Analog of Cardiotonic Steroids'

## Description

### Background of the invention

### 1. Field of the invention

This invention relates to 17-substituted steroids and their use in the treatment of androgen-dependent and oestrogen-dependent disorders, especially prostatic cancer and breast cancer respectively.

### 2. Description of the related art

The 17α-hydroxylase/C₁₇₋₂₀ lyase enzyme complex (hereinafter "hydroxylase/lyase") is known to be essential for the biosynthesis of androgens and oestrogens. In the treatment of androgen-dependent disorders, especially prostatic cancer, there is a need for strong inhibitors of hydroxylase/lyase. Certain anti-androgenic steroids are well known, for example Cyproterone acetate (17α-acetoxy-6-chloro-1α,2α-methylene-4,6-pregnadiene-3,20-dione). Many other steroids have been tested as hydroxylase/lyase inhibitors. See, for example, PCT Specification WO 92/00992 (Schering AG) which describes anti-androgenic steroids having a pyrazole or triazole ring fused to the A ring at the 2,3- position, or European Specifications EP-A 288053 and EP-A 413270 (Merrell Dow) which propose 17β-cyclopropylaminoandrost-5-en-3β-ol or -4-en-3-one and their derivatives.

### Summary of the invention

It has now surprisingly been found that steroids lacking a C₂₀ side chain and having a 17-(3-pyridyl) ring in its place, together with a 16,17-double bond, are powerful hydroxylase/lyase inhibitors, useful for the above-stated purposes.

According to the invention, there are provided compounds of the general formula 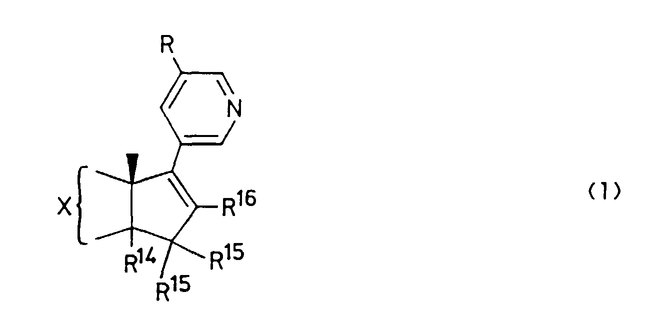 wherein X represents the residue of the A, B and C rings of a steroid, R represents a hydrogen atom or an alkyl group of 1 - 4 carbon atoms, R¹⁴ represents a hydrogen atom, a halogen atom or an alkyl group of 1 to 4 carbon atoms and each of the R¹⁵ substituents independently represents a hydrogen atom or an alkyl or alkoxy group of 1-4 carbon atoms, a hydroxy group or an alkylcarbonyloxy group of 2 to 5 carbon atoms or together represent an oxo or methylene group or R¹⁴ and one of the R¹⁵ groups together represent a double bond and the other R¹⁵ group represents a hydrogen atom or an alkyl group of 1 to 4 carbon atoms, and R¹⁶ represents a hydrogen atom, halogen atom, or an alkyl group of 1 to 4 carbon atoms, in the form of the free bases or pharmaceutically acceptable acid addition salts.

The term "steroid" herein includes any compound having the steroidal B and C rings, but in which all or part of the A ring is missing e.g. ring not closed (lacking the 2- or 3-position C-atom or both) or takes the form of a cyclopentane ring. It also includes azasteroids having a ring nitrogen atom in place of a ring carbon atom, especially in the A-ring such as in 4-azasteroids.

In general, the compounds of formula (1) are new and such compounds per se are included in the invention. However, certain of them have been disclosed as intermediates in the synthesis of certain steroids having a 3-pyridyl or 3-pyridonyl group in the 17β-position, see J. Wicha and M. Masnyk, Bulletin of the Polish Academy of Sciences: Chemistry 33 (1-2), 19-27 and 29-37 (1985). The first of these papers says that a 17β-side chain of the form -C=C-C=0 or -C=C-C=N favours cardiotonic properties and describes the synthesis of 17β-(3-pyridyl)-14β-androst-4-ene-3β,14-diol, while the second uses this compound to prepare 17β-[3 pyrid-2(1H)onyl]-14β-androst-4-ene-3β,14-diol. Those final compounds differ from those of the present invention by having a saturated D-ring and the paper contains no test results. Insofar as certain compounds within formula (1) are known as intermediates in these syntheses, the invention extends to the compounds themselves only for use in therapy.

These are 3β-acetoxy-17-(3-pyridyl)androsta-5,14,16-triene and 3β,15α- and 3β,15β-diacetoxy-17-(3-pyridyl)androsta-5,16-diene. See also J Wicha, et. al., Heterocycles 20, 231-234 (1983) which is a preliminary communication of the first of the above two papers.

J. Wicha et. al., Bulletin of the Polish Academy of Sciences, Chemistry 32 (1-2), 75-83 (1984) have also described the preparation of 3β-methoxy-17β-(3 pyridyl)androstane and pyridone analogues thereof via the intermediate 3β-methoxy-17-(3-pyridyl)-5α-androst-16-ene. Accordingly, the invention extends to the latter compound only for use in therapy. A preliminary communication of this paper, by J. Wicha arid M. Masynk, appeared in Heterocycles 16, 521-524 (1981).

The invention also includes pharmaceutical compositions comprising a compound of formula (1) in association with a pharmaceutically acceptable diluent or carrier. The terminology "pharmaceutical compositions" implies that injectible formulations are sterile and pyrogen-free and thereby excludes any compositions comprising the compound of formula (1) and a non-sterile organic solvent, such as may be encountered in the context of the final stages of preparing these above-mentioned compounds of formula (1) which have been described in the literature but without any therapeutic use being mentioned.

### Description of the preferred embodiments

In the compounds of the invention the essential structural features comprise all of:
- a 3-pyridyl ring in the 17-position
- a ring double bond in the 16,17-position of the D-ring
- the 18-position methyl group

It is critical that the pyridine nitrogen atom be in the 3-position, not the 2- or 4-position. It is also critical that the pyridine ring be joined directly to the 17-carbon atom. This criticality is demonstrated by tests of inhibiting activity against hydroxylase and lyase (Table 1). The concentration of test compound required to achieve 50% inhibition of the enzyme is far greater for the 2-pyridyl, 4-pyridyl and 2-pyridylmethyl compounds tested than for the 3-pyridyl. The methods of determination were as described in the Examples hereinafter.

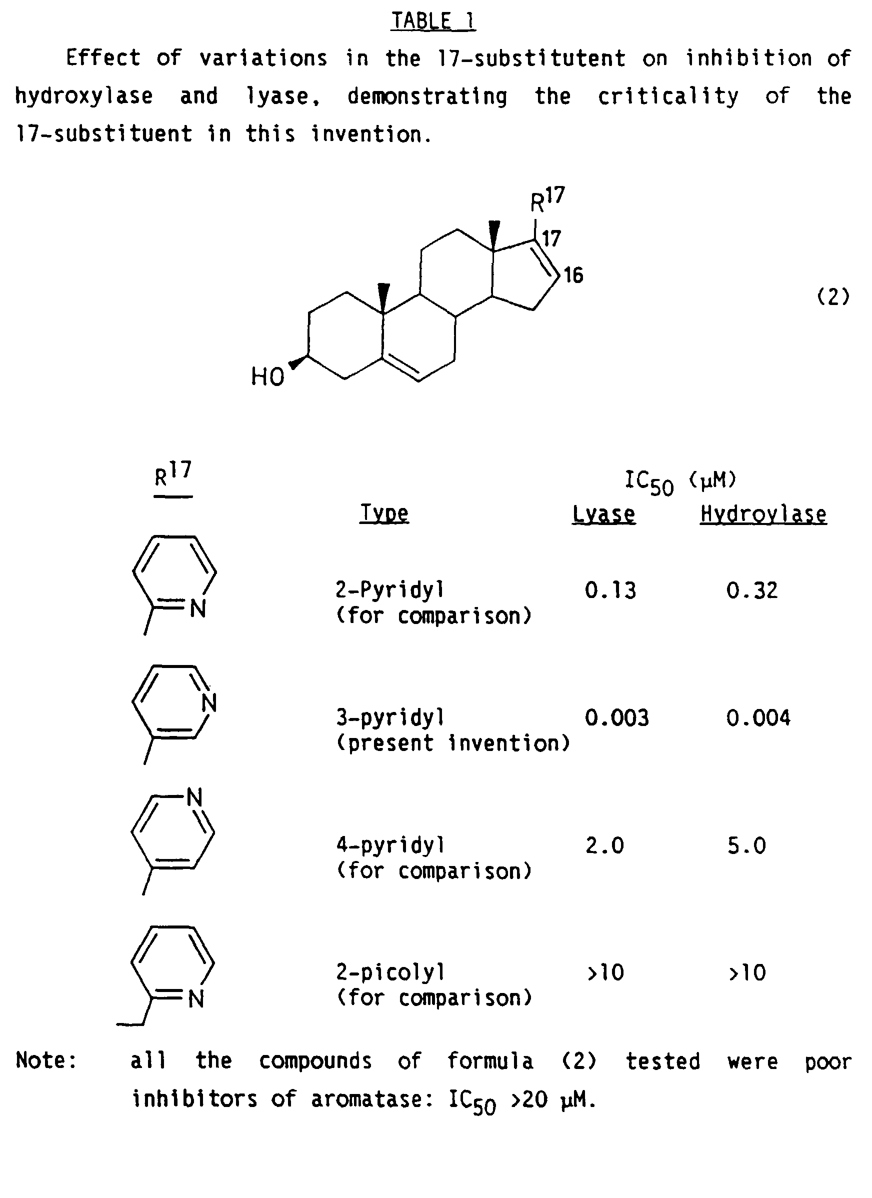

Our modelling of the geometry of the putative transition state of the lyase component of the hydroxylase-lyase enzyme complex, in the putative mechanism of action of the lyase component, suggests that the 16, 17-double bond is essential to allow the 3-pyridine ring to adopt the orientation required for co-ordination to the haem group of the hydroxylase-lyase complex.

Elsewhere, the D-ring can have any other simple substituent. Certain simple substituents are defined in connection with the preferred general formula (1), but it will be appreciated that others could be substituted for those of formula (1). In the compounds of formula (1), R¹⁵ is preferably hydrogen or alkyl of 1 to 3 carbon atoms, R¹⁶ hydrogen, alkyl of 1 to 3 carbon atoms, fluorine, chlorine, bromine or iodine, and R hydrogen or methyl, in the 5-position of the pyridine ring.

The remainder of the molecule, designated "X" in formula (1), can be of any kind conventional in steroid chemistry or have any other feature known in steroids having anti-androgenic activity, for example the pyrazole or triazole ring, fused to the A ring at the 2- and 3- positions, disclosed in the above-cited Specification WO 92/00992, or oxazole ring fused in the same positions.

By way of example, X can represent the residue of
androstan-3α- or 3β-ol,
androst-5-en-3α- or 3β-ol,
androst-4-en-3-one,
androst-2-ene,
androst-4-ene,
androst-5-ene,
androsta-5,7-dien-3α or 3β-ol,
androsta-1,4-dien-3-one,
androsta-3,5-diene,
estra-1,3,5[10]-triene,
estra-1,3,5[10]-trien-3-ol,
5α-androstan-3-one,
androst-4-ene-3,11-dione,
6-fluoroandrost-4-ene-3-one or
androstan-4-ene-3,6-dione
each of which, where structurally permissible, can be further derivatised in one or more of the following ways:
- to form 3-esters, especially 3-alkanoates and -benzoates,
- to have one or more carbon to carbon ring double bonds in any of the 5,6-, 6,7- 7,8-, 9,11- and 11,12-positions
- as 3-oximes
- as 3-methylenes
- as 3-carboxylates
- as 3-nitriles
- as 3-nitros
- as 3-desoxy derivatives
- to have one or more hydroxy, halo, C₁₋₄-alkyl, trifluoromethyl, C₁₋₄-alkoxy, C₁₋₄-alkanoyloxy, benzoyloxy, oxo, methylene or alkenyl substituents in the A, B or C-ring
- to be 19-nor.
   Preferred C₁₋₄-alkyl and alkoxy groups are methyl and ethoxy. Preferred C₁₋₄-alkanoyloxy groups are acetoxy and propanoyloxy.
   Preferred halo groups are fluoro, bromo and chloro and the preferred substitution position is the 6-position.
The substituents include, for instance, 2-fluoro, 4-fluoro, 6-fluoro, 9-fluoro, 3-trifluoromethyl, 6-methyl, 7-methyl, 6-oxo, 7-oxo, 11-oxo, 6-methylene, 11-methylene, 4-hydroxy, 7-hydroxy, 11-hydroxy or 12-hydroxy, each in any appropriate epimeric form, and, subject to structural compatibility (well known in general steroid chemistry), in any combination of two or more such groups.

Compounds which are likely to be unstable are considered excluded from consideration. Such compounds will be evident to steroid chemists. Compounds having esoteric substituents likely to interfere with the stereochemical alignment of the steroid molecule with the enzymes to be inhibited, by virtue of steric or electronic distribution effects, are to be avoided. For example, a 2,3,5,6-tetrafluoro-4-trifluoromethylphenoxy substituent in the 3-position is not recommended. Androst-5-en-3β-ol having such an ether substituent in place of the 3β-hydroxy group has been shown to be a very poor inhibitor for lyase and hydroxylase.

The currently preferred compounds of formula (1) include those which are saturated and unsubstituted at the 11- and 12-positions and which therefore are of the general formula (3): 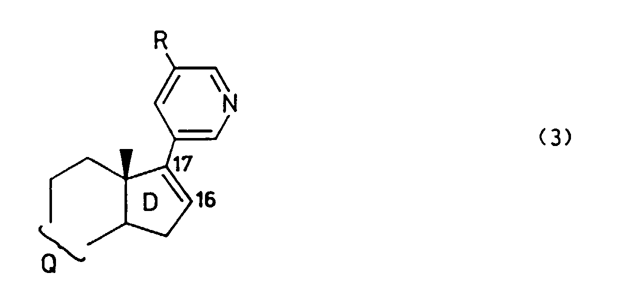 wherein Q represents the residue of A, B and C rings of a steroid, and R is a hydrogen atom or an alkyl group of 1-4 carbon atoms.

However, 11- and/or 12-substituted compounds are also active. Particularly preferred are 11-oxo and 11β-hydroxy derivatives of compounds of formula (3).

Specifically preferred compounds of the invention comprise
17-(3-pyridyl)androsta-5,16-dien-3β-ol,
17-(3-pyridyl)androsta-3,5,16-triene,
17-(3-pyridyl)androsta-4,16-dien-3-one,
17-(3-pyridyl)estra-1,3,5[10],16-tetraen-3-ol,
17-(3-pyridyl)-5α-androst-16-en-3α-ol
and their acid addition salts and 3-esters.

Other notable compounds of the invention comprise
17-(3-pyridyl)-5α-androst-16-en-3-one,
17-(3-pyridyl)-androsta-4,16-diene-3,11-dione,
17-(3-pyridyl)-androsta-3, 5,16-trien-3-ol,
6α-and 6β-fluoro-17-(3-pyridyl)androsta-4,16-dien-3-one
17-(3-pyridyl)androsta-4,16-dien-3,6-dione,
17-13-(5-methyl pyridyl)]androsta-5,16 dien-3β-ol
3α-trifluoromethyl-17-(3-pyridyl)androsta-16-en-3β-ol
and their acid addition salts and 3-esters.

Insofar as certain compounds within formula (1) are known per se and these are compounds which are less easy to prepare than many of the others, a preferred class of compounds of formula (1) is those which do not have a 3β-alkoxy group, a 14, 15-double bond or a 15-ester group.

The compounds of formula (1) can be prepared by a method which is in itself novel and inventive. Starting from a 17-oxo compound of general formula (4): 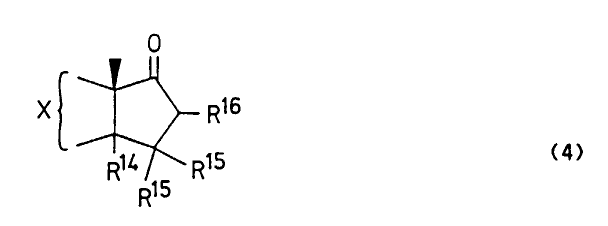 wherein X, R¹⁴, R¹⁵ and R¹⁶ are as defined above and any other oxo groups and hydroxy groups in the molecule are first appropriately protected, the method comprises replacing the 17-hydroxy group of compound (4) in its enol form by a leaving group (L) which is capable of being replaced by a 3-pyridyl group in a palladium complex-catalysed cross-coupling reaction with a pyridyl ring-substituted boron compound of formula (5): 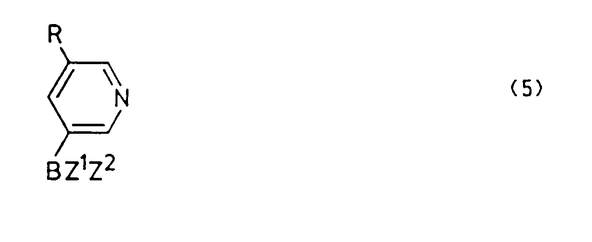 wherein Z¹ and Z² independently represent hydroxy or alkoxy or alkyl of 1-4 carbon atoms each, preferably ethyl or methoxy, and R is as defined above and carrying out said cross-coupling reaction.

The palladium complex-catalysed cross-coupling reaction of the 17-substituted steroid with the boron compound is believed to involve the steps indicated in the following illustrative reaction scheme (Py = 3-pyridyl). The pyridyl anionic species is provided by the boron compound. 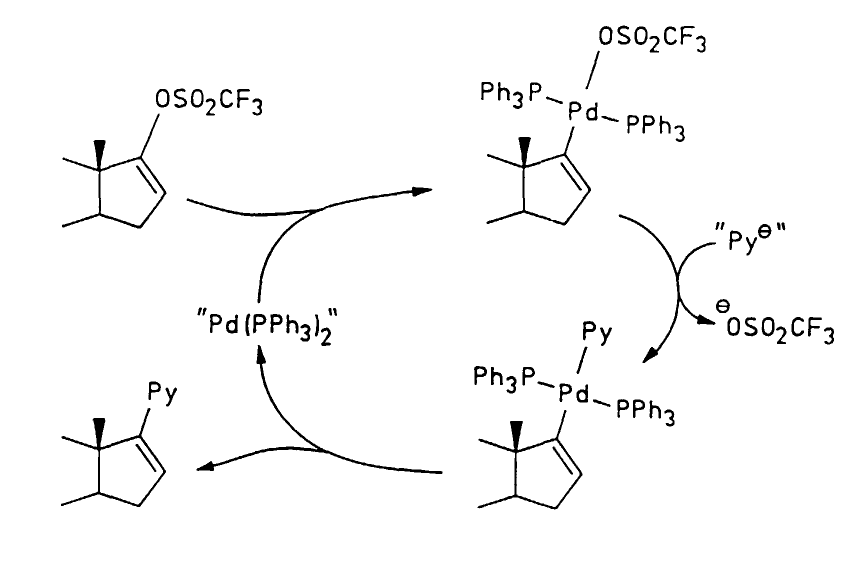 The replacement of the 17-enol group can be, for example, to form a 16,17-ene trifluoromethanesulphonate of formula (6): 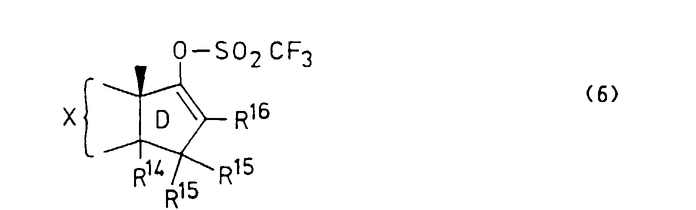 or a 17-iodo or bromo-16,[17]-ene of formula (7): 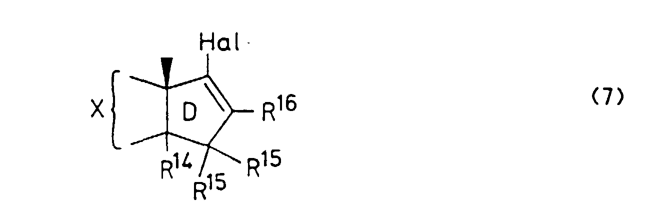 (Hal = I or Br)

Compounds of formula (6) can be prepared by reacting the 17-oxo compound of formula (4) with an enol ester-forming trifluoromethanesulphonic acid derivative such as the anhydride, see S. Cacchi, E. Morera and G. Ortar, Tetrahedron Letters, 25, 4821 (1984). The 17-oxo compound can be considered notionally to exist in the enol form, the reaction being,one of esterification of the enol.

Compounds of formula (7) can be prepared by first hydrazinating the 17-oxo compounds of formula (4) by a standard method to form the 17-hydrazone, which is then reacted with bromine or iodine in the presence of an amine or guanidine base, see D. Barton, G. Bashiardes and J. Fourrey, Tetrahedron Letters, 24, 1605 (1983).

For the preparation of the 17-position derivatives of formula (6) or (7) any necessary protection of other groups in the molecule is first carried out. For example hydroxyl groups are conveniently protected as their acetates, whilst the 3-oxo group of steroids can be selectively protected as their perfluorotolyl enol ethers, see M. Jarman and R. McCague, J.Chem.Soc. Perkin Trans, 1, 1129 (1987).

The 17-position derivative is then reacted with the boron compound of formula (5) using as catalyst a palladium(O) phosphine complex, for example tetrakis(triphenylphosphine)palladium(O), or a palladium (II) phosphine complex which is reducible in situ to a palladium(O) phosphine species, especially bis(triphenylphosphine)palladium (II) chloride.

Further compounds of the invention can be prepared by standard steroid to steroid inter-conversion chemistry, so long as the D-ring chemical structure is not affected thereby. If the D-ring structure is likely to be affected, it would usually be necessary to prepare the desired compound de novo, i.e. by choosing the appropriate starting compound of formula (4), protected if necessary, and carrying out the reactions of 17-substitution of the enol and cross-coupling with the boron compound as described above.

By way of example, the 3-esters of a steroid 3-ol with an alkanoic acid of 1 to 6 carbon atoms, or a cycloalkanoic acid or aralkanoic acid such as phenylacetic or phenylpropionic acid, an aroic acid such as benzoic acid, or other simple organic acid such as methanesulphonic acid, can be converted into the 3-ol or the 3-ol to the 3-ester. Other examples of simple conversions which would not affect the D-ring structure are
i) Oppenauer oxidation using cyclohexanone and aluminium isopropoxide to convert 3-hydroxy to 3-oxo steroids and notably Δ^{5,6}-3-hydroxy to Δ^{4,5}-3-oxo steroids;
ii) Wittig olefination to convert oxo groups to methylene groups [D. D. Evans et al., J. Chem. Soc., 4312-4317, (1963)];
iii) Oxidation of Δ⁵-3β-hydroxy to Δ⁴-3,6-dione steroids using N-methylmorpholine N-oxide and tetra-n-propylammonium perruthenate catalyst [M. Moreno et al., Tetrahedron Letters, 32, 3201-3204, (1991)];
iv) 6-Methylenation of Δ⁴-3-oxo steroids using formaldehyde dimethylacetal [K. Annen et al., Synthesis, 34-40 (1982)];
v) Conversion of Δ⁴-3-oxo to 4,4 dimethyl-Δ⁵-3-oxo, Δ^{1,4}-3-oxo, Δ^{1,4,6}-3-oxo, 7α-methyl-Δ⁴-3-oxo, Δ^{4,6}-3-oxo, 6-chloro-Δ^{4,6}-3-oxo, Δ^{2,4}-2,3-isoxazole, 6α-methyl-Δ⁴-3-oxo and Δ⁴-3-desoxy; Δ⁵-3β-ol to 5α-fluoro-6-oxo. 5α,6,6-trifluoro, 6,6-difluoro and 6α-fluoro-Δ⁴-3-oxo; and 11-oxo to 11-hydroxy and Δ^{9,11} steroids [D. Ladnicer and L. A. Mitscher, The Organic Chemistry of Drug Synthesis, 1s, 2 and 3, Wiley (1980 and 1984)] or
vi) Electrophilic fluorination of steroids using N-fluoropyridiniun reagents [T. Umenoto et al., Organic Synthesis 69, 129 - 143 (1990)].

The compounds of formula (1) may be prepared as salts, e.g. the hydrochloride and converted to the free base form and thereafter to such other conventional pharmaceutically acceptable salts as acetates, citrates and lactates, as may seem appropriate.

The present invention also provides a pharmaceutical composition which comprises a therapeutically effective amount of a compound of the invention, in association with a therapeutically acceptable carrier or diluent. The composition of the invention can, for example, be in a form suitable for parenteral (e.g. intravenous, intramuscular or intracavital), oral, topical or rectal administration. Particular forms of the composition may be, for example, solutions, suspensions, emulsions, creams, tablets, capsules, lipsomes or micro-reservoirs, especially compositions in orally ingestible or sterile injectable form. The preferred form of composition contemplated is the dry solid form, which includes capsules, granules, tablets, pills, boluses and powders. The solid carrier may comprise one or more excipients, e.g. lactose, fillers, disintegrating agents, binders, e.g. cellulose, carboxymethylcellulose or starch or anti-stick agents, e.g. magnesium stearate, to prevent tablets from adhering to tabletting equipment. Tablets, pills and boluses may be formed so as to disintegrate rapidly or to provide slow release of the active ingredient.

Where national patent law permits, the present invention also includes a method of treating androgen- and oestrogen-dependent disorders, especially tumours, and most especially prostatic tumours, in the mammalian body, which comprises administering a compound of the invention to a mammalian patient in a therapeutically effective dose, e.g. in the range 0.001-0.04 mmole/kg body weight, preferably 0.001-0.01 mmole/kg, administered daily or twice daily during the course of treatment. This works out (for humans) at 20-800 mg/patient per day. Alternatively the invention includes the compounds of the invention for use in said treatment and their use in the manufacture of medicaments for that purpose. The preferred use is in treating prostatic cancer. Another use is in treating breast cancer.
The following Examples illustrate the invention.

### Example 1

### (a) 3β-Acetoxyandrosta-5,16-dien-17-yl trifluoromethanesulphonate

To a stirred solution of dehydroepiandrosterone-3-acetate (24.8g, 75 mmol) in dry dichloromethane (500 ml) containing 2,6-di-t-butyl-4-methylpyridine (18.5g, 90 mmol) was added trifluoromethanesulphonic anhydride (12.6 ml, 75 mmol). After 12h the mixture was filtered and washed with water (50 ml), dried (MgSO₄), and the solvent evaporated. Chromatography, on elutlon with light petroleum-dichloromethane (G:1), gave firstly androsta-3,5,16-trien-17-yl trifluoromethanesulphonate (3.02g, 10%) as an oil. ¹H-NMR(CDCl₃) inter alia δ 0.99 (3H,s,18-CH₃), 1.02(3H,s,19-CH₃), 5.39(1H,m,6-H), 5.59(1H,m,16-H), 5.62(1H,m,3-H), 5.93(1H,dm,J 9.4Hz, 4-H); MS m/z 402(M⁺). Further elution with light petroleum-dichloromethane (3:1) afforded the title compound (20.1g, 58%) which crystallised from hexane, m.p. 75-76°C. ¹H-NMR(CDCl₃) inter alia δ 1.00(3H,s,18-CH₃), 1.06(3H, s,19-CH₃), 2.04(3H,s,CH₃CO₂), 4.59(1H,m,3α-H), 5.39(1H,dm,J 4.9 Hz, 6-H), 5.58(1H,m,16-H). Anal. Calcd: C,57.13; H,6.32; 5,6.93. Found: C,57.29; H,6.31; S, 6.96%.

### (b) 3β-Acetoxy-17-(3-pyridyl)androsta-5,16-diene

Diethyl(3-pyridyl)borane (3.38g, 23 mmol) from Aldrich Chemical Co. Ltd. was added to a stirred solution of 3β-acetoxyandrosta-5,16-dien-l7-yl trifluoromethanesulphonate (6.94g, 15 mmol) in THF (75 ml) containing bis(triphenylphosphine)palladium(II) chloride (0.105g, 0.15 mmol). An aqueous solution of sodium carbonate (2M, 30 ml) was then added and the mixture heated, with stirring, by an oil bath at 80°C for lh, and allowed to cool. The mixture was partitioned between diethyl ether and water, the ether phase was dried (Na₂CO₃), filtered through a short plug of silica, and concentrated. Chromatography, on elution with light petroleum-diethyl ether (2:1), afforded the title compound (4.95g, 84%) which crystallised from hexane, m.p. 144-145°C, ¹H-NMR(CDCl₃) inter alia δ 1.05(3H,s,19-CH₃), 1.08(3H,s,18-CH₃), 2.04(3H,s,CH₃CO₂), 4.60(1H,m,3α-H), 5.42(1H,dm, J 4.7Hz,6-H), 5.99(1H,m,16-H), 7.23(1H,m,Py 5-H) 7.65(1H,m,Py 4-H), 8.46(1H,m,Py 6-H), 8.62(1H,m,Py 2-H). Anal. Calcd: C, 79.75; H, 8.50; N, 3.58. Found: C, 79.78; H, 8.52; N, 3.54%.

### Example 2

### 17-(3-Pyridyl)androsta-5,16-dien-3β-ol

To a solution of 3β-acetoxy-17-(3-pyridyl)androsta-5,16-diene (4.90g, 12.5 mmol) in methanol (50 ml) was added an aqueous solution of sodium hydroxide (10% w/v, 10 ml) and the mixture heated, with stirring, on an oil bath at 80°C for 5 min., then allowed to cool. The mixture was poured into water, neutralised with hydrochloric acid (1M), rebasified with saturated sodium bicarbonate solution, and extracted with hot toluene (3 x 100 ml). The toluene extracts were combined, dried (Na₂CO₃), and concentrated. Chromatography, on elution with toluene-diethyl ether (2:1) afforded the title compound (3.45g, 79%) which crystallised from toluene, mp 228-229°C; ¹H-NMR (CDCl₃ inter alia δ 1.05(3H,s,19-CH₃), 1.07(3H,s,18-CH₃), 3.54(1H,m,3α-H), 5.40(1H,dm, J 5.0 Hz, 6-H), 5.99(1H,m,16-H), 7.22(1H,m,Py5-H), 7.65(1H,m,Py 4-H), 8.46(1H,m,Py 6-H), 8.62(1H,m,Py 2-H). Anal. Calcd: C, 82.47; H, 8.94; N, 4.01. Found: C, 82.40; H, 8.91; N, 3.97%.

### Example 3

### 17-(3-Pyridyl)androsta-3,5,16-triene

The method followed that described in Example 1, using in step (b) diethyl(3-pyridyl)borane (0.88g, 6.0 mmol), androsta-3,5,16-trien-17-yl trifluoromethanesulphonate (2.01g, 5.0 mmol), prepared in step (a), THF (25 ml), bis(triphenylphosphine)palladium(II) chloride (35 mg, 0.05 mmol), and aqueous sodium carbonate (2M, 10 ml). Chromatography, on elution with dichloromethane, afforded the title compound (1.39g, 84%) which crystallised from hexane, m.p. 110-112°C. ¹H-NMR (CDCl₃) inter alia δ 1.02(3H,s,19-CH₃), 1.07(3H,s,18-CH₃), 5.44(1H,m,6-H), 5.61(1H,m,3-H), 5.95(1H,dm, J 9.8Hz, 4-H), 6.01(1H,m,16-H), 7.23(1H,m,Py 5-H), 7.66(1H,m,Py 4-H), 8.46(1H,m,Py 6-H), 8.63(1H,m,Py 2-H); MS m/z 331 (M⁺).

### Example 4

### (a) 3-[2,3,5,6-tetrafluoro-4-(trifluoromethyl)phenoxylandrosta-3,5,16-trien-17-yl trifluoromethanesulphonate

The method followed that described in Example 1(a) but using 3-[2,3,5,6-tetrafluoro-4-(trifluoromethyl)phenoxy]androsta-3,5-dien-17-one (5.03g, 10 mmol), prepared as described in M. Jarman and R. McCague, J. Chem. Soc., Perkin Trans. 1, 1129 (1987), dichloromethane (80 ml), 2,6-di-t-butyl-4-methylpyridine (2.87g, 14 mmol), and trifluoromethanesulphonic anhydride (1.85 ml, 11 mmol). Chromatography, on elution with light petroleum-dichloromethane (10:1), afforded the title compound (1.93g, 30%) which crystallised from ethanol, m.p. 106-107°C. ¹H-NMR (CDCl₃) inter alia δ 1.02(6H,s,18 and 19-CH₃), 5.16(1H,s,4-H), 5.28(1H,m,6-H), 5.59(1H,m,16-H); MS m/z 634 (M⁺).

### (b) 3-[2,3,5,6-tetrafluoro-4-(trifluoromethyl)phenoxy]-17-(3-pyridyl)androsta-3,5,16-triene

The method essentially followed that of Example 1(b) but using diethyl(3-pyridyl)borane (0.44g, 3.0 mmol), 3-12,3,5,6-tetrafluoro-4-(trif1uoromethyl)phenoxy]androsta-3,5,16-trien-17-yl trifluoromethanesulphonate (1.27g, 2.0 mmol), THF (10 ml), bis(triphenylphosphine)palladium(II) chloride (70mg, 0.1 mmol), and aqueous sodium carbonate (2M, 5 ml). Chromatography, on elution with light petroleum-diethyl ether (3:1), afforded the title compound (0.82g, 73%) which crystallised from hexane, m.p. 166.0-166.5°C. ¹H-NMR (CDCl₃) inter alia δ 1.05(3H,s,19-CH₃), 1.07(3H,s,18-CH₃), 5.18(1H,s,4-H), 5.32(1H,m,6-H), 6.01(1H,m,16-H), 7.23(1H,m,Py 5-H), 7.66(1H,m,Py 4-H), 8.47(1H,m,Py 6-H), 8.63(1H,m,Py 2-H). Anal. Calcd: C, 66.07; H, 5.01; N, 2.49; F, 23.60. Found: C, 65.97; H, 5.02; N, 2.47; F, 23.41%.

### (c) 17-(3-Pyridyl)androsta-4,16-dien-3-one

To a solution of 3-[2,3,5,6-tetrafluoro-4-(trifluoromethyl)-phenoxy]-17-(3-pyridyl)androsta-3,5,16-triene (0.423g, 0.75 mmol) in THF (5 ml) was added ethanol (5 ml) followed by aqueous hydrochloric acid (1M, 5 ml) and the mixture heated, with stirring, by an oil bath at 65°C for 48h and allowed to cool. The mixture was poured into water (20 ml), neutralised with aqueous sodium hydroxide (1M), and extracted with diethyl ether (3 x 30 ml). The ether extracts were combined, dried (Na₂CO₃), and concentrated. Chromatography, on elution with diethyl ether, afforded the title compound (185mg, 71%) which crystallised from diethyl ether, m.p. 148-150°C. IR vmax 1674 cm⁻¹; ¹H-NMR(CDCl₃) inter alia δ 1.07(3H,s,18-CH₃), 1.24(3H,s,19-CH₃), 5.76(1H,s,4-H), 5.99(1H,m,16-H), 7.23(1H,m,Py 5-H), 7.64(1H,m,Py 4-H), 8.47(1H,m,Py 6-H), 8.62(1H,m,Py 2-H); MS m/z 347 (M⁺).

### Example 5

### (a) 3-Acetoxyestra-1,3,5[10],16-tetraen-17-yl trifluoromethanesulphonate

The method followed that described in Example 1(a), but using oestrone-3-acetate (4.69g, 15 mmol). dichloromethane (120 ml), 2,6-di-t-butyl-4-methylpyridine (4.00g, 19.5 mmol), and trifluoromethanesulphonic anhydride (2.8 ml, 16.5 mmol). Chromatography, on elution with light petroleum-dichloromethane (3:1), afforded the title compound (5.21g, 78%). ¹H-NMR(CDCl₃) inter alia δ 1.00(3H,s,18-CH₃), 2.29(3H,s,CH₃CO₂), 5.62(1H,m,16-H), 6.81(1H,m,ArH), 6.85(1H,m,ArH), 7.26(1H,m,ArH). Anal. Calcd. for C₂₁H₂₃O₅F₃S₁.½H₂O: C, 55.62; H, 5.34. Found: C, 55.58: H, 5.14%.

### (b) 3-Acetoxy-17-(3-pyridyl)estra-1,3,5[10],16-tetraene

The method followed that described in Example 1(b), but using diethyl(3-pyridyl)borane (1.65g, 11.2 mmol), 3-acetoxyestra-1,3,-5[10],16-tetraen-17-yl trifluoromethanesulphonate (3.56g, 8.0 mmol), THF (40 ml), bis(triphenylphosphine)palladium(II) chloride (56mg, 0.08 mmol), and aqueous sodium carbonate (2M, 15 ml).
Chromatography, on elution with light petroleum-diethyl-ether (2:1) afforded the title compound (2.40g, 80%). ¹H-NMR(CDCl₃) inter alia δ 1.04(3H, s,18-CH), 2.29(3H, s, CH₃CO₂), 6.03(1H,m,16-H), 6.82(1H,m,ArH), 6.85(1H,m,ArH), 7.24(1H,m,Py 5-H), 7.29(1H,m,ArH), 7.69(1H,m,Py 4-H), 8.48(1H,m,Py 6-H), 8.65(1H,m,Py 2-H); MS m/z 373. (M⁺).

### Example 6

### 17-(3-Pyridyl)estra-1,3,5[10],16-tetraen-3-ol

The method followed that described in Example 2, but using 3-acetoxy-17-(3-pyridyl)estra-1,3,5[10],16-tetraene (2.36g, 6.3 mmol), methanol (40 ml), aqueous sodium hydroxide (10% w/v, 5 ml), and the mixture was heated at 80°C for 10 min. Chromatography, on elution with toluene-methanol (8:1), afforded the title compound (1.40g, 67%) which crystallised from toluene, m.p. 256-258°C: ¹H-NMR(DMSO) inter alia δ 1.01(3H,s,18-CH₃), 6.15(1H,m,16-H), 6.47(1H,m,ArH), 6.52(1H,m,ArH), 7.04(1H,m,ArH), 7.35(1H,m,Py 5-H), 7.79(1H,m,Py 4-H), 8.45(1H,m,Py 6-H), 8.62(1H,m,Py 2-H). Anal. Calcd: C, 83.34; H, 7.60; N, 4.23. Found: C, 83.39; H, 7.78; N, 4.06%.

### Example 7

### 3α-Acetoxy-17-(3-pyridyl)-5α-androst-16-ene

The method followed that described in Example 1, using in step (b) diethyl(3-pyridyl)borane (1.41g, 9.6 mmol), 3α-acetoxy-5α-androst-16-en-17-yl trifluoromethanesulphonate (3.44g, 7.4 mmol), prepared from the 3α-acetoxy-5α-androstan-17-one by the method of step (a), THF (40 ml), bis(triphenylphosphine)palladium(II) chloride (52 mg, 0.07 mmol), and aqueous sodium carbonate (2M, 15 mmol). Chromatography, on elution with light petroleum-diethyl ether (2:1), afforded the title compound (2.39g, 82%), ¹H-NMR (CDCl₃) inter alia δ 0.85(3H,s,19-CH₃), 1.01(3H,s,18-CH₃), 2.06(3H,s,CH₃CO₂), 5.02(1H,m,3β-H), 6.00(1H,m,16-H), 7.24(1H,m,Py 5-H), 7.68(1H,m,Py 4-H), 8.47(1H,m,Py 6-H), 8.63(1H,m,Py 2-H); MS m/z 393 (M⁺).

### Example 8

### 17-(3-Pyridyl)-5α-androst-16-en-3α-ol

The method followed that described in Example 2, but using 3α-acetoxy-17-(3-pyridyl)-5α-androst-16-ene (2.33g, 5.9 mmol), methanol (40 ml), aqueous sodium hydroxide (10% w/v, 8 ml), and the mixture was heated at 80°C for 20 min. Chromatography, on elution with toluene-methanol (40:1), afforded the title compound (1.62g, 78%) which crystallised from toluene, m.p. 198-199°C; ¹H-NMR(CDCl₃) inter alia. δ 0.84(3H,s,19-CH₃), 1.00(3H,s,18-CH₃), 4.06(1H,m,3β-H), 5.97(1H,m,16-H), 7.21(1H,m,Py 5-H), 7.64(1H,m,Py 4-H), 8.45(1H,m,Py 6-H), 8.61(1H,m,Py 2-H). Anal. Calcd: C, 82.00; H,9.46; N,3.99. Found: C,81.78; H,9.47; N.3.96%.

### Example 9

### 17-(3-Pyridyl)-5α-androst-16-en-3-one

From a solution of 17-(3-Pyridyl)-5α-androst-16-en-3α-ol (1.05g, 3.0 mmol) in dry toluene (60ml) and cyclohexanone (10ml) was distilled off part of the solvent (20ml) to eliminate moisture. After allowing to cool to 90°C, aluminium isopropoxide (1.02g, 5.0 mmol) was added and the mixture heated under reflux for 90 min. then allowed to cool. The mixture was diluted with diethyl ether (250 ml), washed with aqueous trisodium citrate (15% w/v; 2 x 30ml), dried (Na₂CO₃), and concentrated. Chromatography, on elution with toluene-methanol (40:1), afforded the title compound (0.90g, 86%) which crystallised from toluene, m.p. 190-192°C. IR νmax 1713 cm⁻¹; ¹H-NMR (CDCl₃) inter alia δ 1.04 (3H,s,19-CH₃), 1.07 (3H,s,18-CH₃), 5.98 (1H,M,16-H), 7.22 (1H,m,Py 5-H), 7.64 (1H,m,Py 4-H), 8.46 (1H,m,Py 6-H), 8.61 (1H,m,Py 2-H); MS m/z 349 (M+). Anal. Calcd: C,82.47; H,8.94; N,4.01. Found: C,82.00; H,8.94; N,3.84%.

### Example 10

### a) 3-(tert-Butyldimethylsiloxy)androsta-3,5-diene-11,17-dione

To a solution of adrenosterone (6.0g, 20 mmol) in dry dichloromethane (120ml) was added triethylamine (8.4ml, 60 mmol) followed by tert-butyldimethylsilyl trifluoromethanesulfonate (5.0ml, 22 mmol) and the mixture stirred at room temperature for 3h. The dichloromethane was evaporated and the residue redissolved in diethyl ether (100ml), then allowed to stand for 30 min, after which time an oil separated. The ether phase was decanted off the oil and the solvent evaporated to give the title compound which was used directly in step (b). IR νmax 1705, 1747 cm⁻¹; ¹H-NMR(CDCl₃) inter alia δ0.12 (6H,s,Me₂Si), 0.85 (3H,s,18-CH₃), 0.92 (9H,s,^{t}BuSi) 1.17(3H,s,19-CH₃), 4.73 (1H,dm, J 6.9Hz, 6-H), 5.36 (1H,m,4-H).

### b) 13-(tert-Butyldimethylsiloxy)-11-oxo-androsta-3,5,16-trien-17-yl trifluoromethanesulfonate

To a solution of the product from step (a) in dry THF (100ml), cooled to -78°C, was added a freshly prepared solution of lithium diisopropylamide [prepared by adding n-butyllithium (1.6M; 13.8ml, 22 mmol) in hexane to a solution of diisopropylamine (3.lml, 22 mmol) in dry THF (25ml) at -18°C] and the resultant yellow solution stirred at -78°C for 30 min. A solution of N-phenyltrifluoromethanesulfonimide (7.15g, 20 mmol) in dry THF (20ml) was then added and after an additional lh. at -78°C was allowed to reach ambient temperature. The reaction mixture was poured into water (200 ml) and extracted with diethyl ether (2 x 200ml), the combined ether extracts were washed with water (20ml), dried Na₂CO₃), and concentrated to give the title compound which was used directly in step (c). IR νmax 1710 cm⁻¹, ¹H-NMR (CDCl₃) inter alia δ0.13 (6H,S,Me₂Si), 0.92 (9H,s, ^{t}Bu Si), 1.35 (6H,2s,18-CH₃ and 19-CH₃), 4.75 (1H,m,6-H) 5.38 (1H,s,4-H), 5.68 (1H,m,16-H).

### c) 3-(tert-Butyldimethylsiloxy)-17-(3-pyridyl)androsta-3,5,16 -trien-11-one

The method essentially followed that described in Example 1 (b), but using the 13-(tert-butyldimethylsiloxy)-11-oxo-androsta-3,5,16-trien-17-yl trifluoromethanesulfonate from step (b), diethyl (3-pyridyl)borane (3.53g. 24 mmol), THF (100ml), bis(triphenylphosphine)palladium (II) chloride (280mg, 0.4 mmol), and aqueous sodium carbonate (2M;50ml). Following work-up as described in Example 1 (b) the title compound was obtained, which was used directly in step (d). IR νmax 1705 cm⁻¹, ¹H-NMR (CDCl₃) inter alia δ0.13 (3H,s,Me₂Si), 0.93 (9H,s,^{t}BuSi), 0.99 (3H,s,18-CH₃), 1.18 (3H,s,19-CH₃), 4.75 (1H,m,6-H) 5.37 (1H,m,4-H), 6.09 (1H,m,16-H), 7.26 (1H,m,Py 5H), 7.62 (1H,m,Py 4-H), 8.50 (1H,m,Py 6-H), 8.60 (1H,m,Py 2-H). MS m/z 475 (M+).

### d) 17-(3-Pyridyl)androsta-4,16-diene-3,11-dione

To a solution of the product from step (c) in wet THF (60ml) was added a solution of tetrabutylammonium fluoride (1.0N; 10ml, 10 mmol) in THF, and the mixture stirred at room temperature for 12 h. The mixture was partitioned between diethyl ether and water basified with saturated aqueous sodium bicarbonate, the ether phase isolated, dried (Na₂CO₃), and concentrated. Chromatography, on elution with diethyl ether, afforded the title compound (4.30g, 60% overall yield from adrenosterone) which crystallised from diethyl ether, m.p. 181-183°C.
IR νmax 1669, 1703 cm⁻¹, ¹H-NMR(CDCl₃) inter alia δ1.02 (3H,s, 18-CH₃), 1.45 (3H,s,19-CH₃), (1H,(1H,S,Py 4-H), 6.08 (1H,m,16-H) 7.24 (1H,m,Py 5-H), 7.59 (1H,m,Py 4-H),8.50 (1H,m,Py 6-H), 8.59 (1H,m,Py 2-H). MS m/z 361 (M+). Anal Calcd: C, 79.74; H,7.53: N,3.88. Found: C,79.58; H,7.57; N,3.89%.

### Example 11

### 3-Acetoxy-17-(3-pyridyl)androsta-3,5,16-triene

17-(3-pyridyl)androsta-4,16-dien-3-one (174 mg, 0.50 mmol) was dissolved in isopropenyl acetate (2 ml). p-Toluenesulfonic acid (130 mg, 0.68 mmol) was then added and the mixture heated at 80°C for 12h. After allowing to cool the mixture was poured into diethyl ether, washed with saturated aqueous sodium bicarbonate, dried (Na₂CO₃) and concentrated. Chromatography on elution with light petroleum - diethyl ether (1:1), afforded the title compound (86 mg, 44%), IR νmax 1755 cm⁻¹, ¹H-NMR (CDCl₃) inter alia δ1.05 (6H,s,18-CH₃ and 19-CH₃), 2.15 (3H,s,COCH₃) 5.44 (1H,m,6-H), 5.72(1H,m,4-H), 6.00 (1H,m,16-H), 7.25 (1H,m,Py 5-H), 7.66 (1H,m,Py 4-H), 8.47 (1H,M,Py 6-H), 8.63 (1H,m,Py 2-H). MS m/z 389 (M+).

### Example 12

### 6β-Fluoro-17-(3-pyridyl)androsta-4,16-dien-3-one and

### Example 13

### 6α-Fluoro-17-(3-pyridyl)androsta-4,16-dien-3-one

To a solution of 3-acetoxy-17-(3-pyridyl)androsta-3,5,16-triene (80mg, 0.21 mmol) in dry dichloromethane (2ml) was added N-fluoropyridinium trifluoromethanesulfonate (180mg, 0.73 mmol) and the mixture heated under reflux for 12h. The mixture was diluted with diethyl ether (30ml), washed with dilute aqueous sodium hydroxide (0.5M; 2 x 5ml), dried Na₂CO₃), and concentrated. ¹H and ¹⁹F-NMR at this stage showed the 6-fluorinated products were formed as a 3:2 mixture of the β and α-epimers. Chromatography, on elution with light petroleum-diethyl ether (1:2), gave firstly:- i) the title 6β-epimer (13mg), 17%) as white crystals, m.p. 167-169°C. IR νmax 1684 cm⁻¹; ¹H-NMR (CDCl₃) inter alia δ1.11 (3H,s,18-CH₃), 1.37 (3H,s,19-CH₃), 5.06 (1H,dd, J_{H}-_{H} 2.4 Hz, J_{H}-_{F} 49Hz, 6α-H), 5.92 (1H,m,4-H), 6.01 (1H,m,16-H), 7.24 (1H,m,Py 5-H), 7.65 (1H,m,Py 4-H), 8.48 (1H,m,Py 6-H), 8.63 (1H,m,Py 2-H). ¹⁹F-NMR δ -165.9 (dt, J_{H-F} 49 Hz, 6β-F)- MS m/z 365 (M+).
Further elution afforded:-
ii) The title 6α-epimer (8mg, 11%) as white crystals, m.p. 167-169°C, IR νmax 1681 cm⁻¹; ¹H-NMR (CDCl₃) inter alia δ1.07 (3H,s,18-CH₃), 1.24 (3H,s,19-CH₃), 5.18 (1H,dm, J_{H}-_{F} 48Hz, 6β-H), 5.98 (ZH,m,4-H and 16-H), 7.26 (1H,m,Py 5-H), 7.64 (1H,m,Py 4-H), 8.40 (1H,m,Py6-H), 8.63 (1H,m,Py 2-H). ¹⁹F-NMR (CDCl₃) δ -183.9 (d, J_{H-F} 48 Hz, 6α-F). MS m/z 365 (M+).

### Example 14

### 17-(3-pyridyl)androsta-4,16-dien-3-one (via Oppenauer Oxidation)

This Example illustrates a better method of preparing the compound already prepared in Example 4. The method followed that described in Example 9, but using 17-(3-pyridyl)androsta-5, 16-dien-3β-ol (1.05g, 3.0 mmol). Chromatography, on elution with toluene-methanol (20:1), afforded the title compound (0.85g, 82%), which crystallised from diethyl ether, m.p. 148-150°C. Spectroscopic data was identical to that given in Example 4(c).

| | | | |
|---|---|---|---|
| Anal. Calcd | C,82.95; | H,8.41; | N,4.03 |
| Found | C,83.00; | H,8.50; | N,3.99% |

### Example 15

### 17-(3-pyridyl)androsta-4,16-dien-3-one oxime

To a suspension of 17-(3-pyridyl)androsta-4,16-dien-3-one (125 mg, 0.36 mmol) in ethanol (2 ml) was added hydroxylamine hydrochloride (50mg, 0.72 mmol), followed by pyridine (0.2ml), and the mixture heated under reflux for 1h. then allowed to cool.

The solvent was evaporated and the crystalline product triturated under water, collected on a sinter, washed with cold water, and dried in vacuo to give the title oxime as a 1:1 mixture of syn and anti geometric isomers. ¹H-NMR (CDCl₃) inter alia δ1.06 (3H,s,18-CH₃), 1.13 (3H,s,19-CH₃), 5.75 and 5.80 (1H,2m, isomeric 4-H), 6.01 (1H,m,16-H), 7.26 (1H,m,Py 5H), 7.68 and 7.88 (1H, 2m, isomeric Py 4-H), 8.48 and 8.53 (1H, 2m, isomeric Py 6-H), 8.63 (1H,m,Py 2-H). MS m/z 362 (M+).

### Example 16

### 17-(3-pyridyl)androsta-4,16-diene-3,6-dione

To a solution of 17-(3-pyridyl)androsta-5,16-dien-3β-ol (350mg, 1.0 mmol) in dry dichloromethane (10 ml) was added N-methylmorphine N-oxide (351mg, 3.0 mmol) followed by 400mg of freshly dried and powdered 4Å molecular sieves and the mixture stirred for 10 min. Tetrapropylammonium perruthenate catalyst (35mg), 0.1 mmol) was then added, the reaction flask placed in an ultrasonic bath, and the mixture irradiated whilst maintaining the temperature between 20-30°C for 2 h. The mixture was then filtered, diluted with diethyl ether, washed with water, dried (Na₂CO₃), and concentrated. Chromatography, on elution with diethyl ether - ethyl acetate (5:1), afforded the title compound (26 mg, 7%) as white crystals m.p. 210-212°C. IR νmax 1680 cm⁻¹; ¹H-NMR (CDCl₃) inter alia δ1.10 (3H,s,18-CH₃), 1.44 (3H,s,19-CH₃), 4.42 (1H,m,enolic 2-H), 5.84 (1H,s,4-H), 6.01 (1H,m,16-H), 7.24 (1H,m,Py 5-H), 7.65 (1H,m,Py 4-H), 8.45 (1H,m,Py 4-H), 8.45 (1H,m,Py 6-H), 8.60 (1H,m,Py 2-H). FAB-MS MS m/z 362 (M+1).

### Example 17

### 3α-(Trifluoromethyl)-17-(3-pyridyl)androst-16-en-3β-ol

To a solution of 17-(3-pyridyl)androst-16-en-3-one (100 mg, 0.29 mmol) in THF (2ml) cooled to 0°C was added trifluoromethyltrimethylsilane (200µl , 1.3mmol) followed by tetrabutylammonium fluoride trihydrate (10 mg, 0.03 mmol). After 30 min., dilute aqueous hydrochloric acid (1M; 1ml) was added and the mixture stirred at room temperature for 12h. The mixture was then basified with saturated aqueous sodium bicarbonate and extracted with diethyl ether. The three extracts were combined, dried (Na₂CO₃), and concentrated. Chromatography, on elution with light petroleum - diethyl ether (1:1), afforded the title compound (87mg, 73%) which crystallised from toluene, m.p. 192-193°C ¹H-NMR (CDCl₃) inter alia δ0.92 (3H,s,19-CH₃), 1.01 (3H,s,18-CH₃), 5.98 (1H,m,16-H), 7.22 (1H,m,Py 5-H), 7.64 (1H,m,Py 4-H),8.45 (1H,m,Py 6-H), 8.60 (1H,m,Py 2-H); ¹⁹F-NMR (CDCl₃) δ -79.1 (s,3α-CF₃). MS m/z 419 (M+).

| | | | | |
|---|---|---|---|---|
| Anal. Calcd | C,71.57; | H,7.69; | N,3.34; | F,13.59 |
| Found | C,71.67; | H,7.71; | N,3.25; | F,13.30% |

### Example 18

### (a) Diethyl[3-(5-methylpyridyl)] borane

3-Bromo-5-methylpyridine, which can be prepared as described in the literature, e.g. L. van der Does and H. J. van Hertog, Rec. Trav. Chem. Pays Bas 84, 957-960 (1985) or R. A. Abramovitch and M. Saha, Can. J. Chem. 44, 1765-1771 (1966), is reacted with n-butyllithium, according to the method of M. Terashima et al., Chem. Pharm. Bull. 31, 4573-4577, (1983). The product is treated with triethylborane and then iodine.

### (b) 17-[3-(5-Methylpyridyl)]androsta-5,16-dien-3β-ol

Diethyl [3-(5-methylpyridyl)] borane is reacted with 3β-acetoxyandrosta-5,16-dien-17-yl trifluoromethane sulphonate analogously to Example 1 (b) and the resulting 3β-acetate is hydrolysed with sodium hydroxide, analogously to Example 2, to yield the title compound.

### Test results

### (a) Preparation of testicular material

Human testes were obtained from previously untreated patients undergoing orchidectomy for prostatic cancer. The testes were decapsulated and stored in liquid nitrogen until use. A microsomal preparation was prepared essentially as described by S. E. Barrie et al., J. Steroid Biochem. 6, 1191-5, (1989). The material was then thawed, finely chopped, and homogenised in 0.25M sucrose (5ml/g wet weight) using a Potter homogeniser. The homogenate was centrifuged at 12000g for 30 min, and then the microsomes were pelleted by spinning the supernatant at 100,000g for lhr. The pellet was washed by being resuspended in 0.25M sucrose and repelleted. The microsomal pellet was then resuspended in 50mM sodium phosphate pH 7.4/glycerol (3/1 v/v) and stored in aliquots in liquid nitrogen.

### (b) Determination of 17α-hydroxylase

The basic assay mixture was EDTA (0.2mM), dithiothreitol (DTT; 0.1mM), NADPH (0.25mM), glucose 6-phosphate dehydrogenase (G6PDH; 6.25 µg/ml), MgCl₂ (1mM), glucose 6-phosphate (G6P; 10mM) and the substrate, ³H-progesterone (3µM) in sodium phosphate (50mm), pH 7.4. The compounds under test were dissolved in 50% DMSO and the final concentrations of ethanol and DMSO were 1% each. The assay reaction was carried out for 1 hour and was terminated by the addition of 2 vols. of methanol-acetonitrile (2:1) containing approx. 100µM unlabelled progesterone, 17α-hydroxyprogesterone, androstenedione, testosterone, and 16α-hydroxyprogesterone. The last-mentioned steroid was added as it appeared that the human enzyme was capable of 16α-hydroxylation as well as 17α-hydroxylation.

The separation of the steroids by HPLC was carried out using an "Uptight" guard column packed with 40-63µm Nucleosil C18 and a 10cm main column packed with 5µm Nucleosil C18 and 60% methanol as eluant. The radioactivity in the peaks of interest was monitored on-line by mixing the HPLC effluent 1:1 with Ecoscint A (National Diagnostics) scintillation fluid, containing 25% acetonitrile, and passing the mixture through a Berthold LB506C radiochemical monitor. The hydroxylase activity was measured as the production of 17α-hydroxyprogesterone, androstenedione and testosterone.

### (c) Determination of C₁₇-C₂₀ lyase

The mixture was the same as described above for the 17«-hydroxylase except that the substrate was ³H-17α-hydroxyprogesterone. The reaction was carried out for 1-2h. and was stopped by the addition of 2 vols. of methanol/acetonitrile (2/1 containing approx. 100µM 17α-hydroxyprogesterone, androstenedione and testosterone.

The HPLC separation used for the lyase involved a mini-re-column "Uptight Guard Column" packed with PELL-ODS (pellicular octadecyl silica) and a 10cm. main column "Apex C18" column packed with 5µ APEX-CAT silica.
The eluant was 38:12:50 methanol:acetonitrile:water flowing at lml/min. The effluent was mixed 1:1 with Ecoscint A containing 5% methanol and 5% acetonitrile and the radioactivity was measured directly by a Berthold LB506C radiochemical detector. The lyase activity was measured as the production of androstenedione and testosterone.

### (d) Calculation of IC₅₀.

The enzyme activity was measured in the presence of at least 4 concentrations of each compound. The data were for the 4-pyridyl and 2-picolyl compounds of Table 1 fitted by linear regression to the Dixon equation (M. Dixon, E.C. Webb, Enzymes, 2nd ed., Academic Press, New York, 1964). Data for all the other compounds were fitted by non-linear regression to the median effect equation of Chou, J. Theoret. Biol. 39, 253-276 (1976). The correlation coefficients were greater than 0.95 except for the compound of Example 1, where it was 0.91. All the assays were carried out with approx. 4nM enzyme (as calculated from kinetic measurements) except those for Ketoconazole and the 2- and 4-pyridyl and 2-picolyl compounds of Table 1, in which 25nM lyase and 10nM hydroxylase were used. The IC₅₀ values are dependent on enzyme concentration when the inhibitor binds tightly (all the compounds tested except the 4-pyridyl and 2-picolyl). Results are shown in Table 2 below.

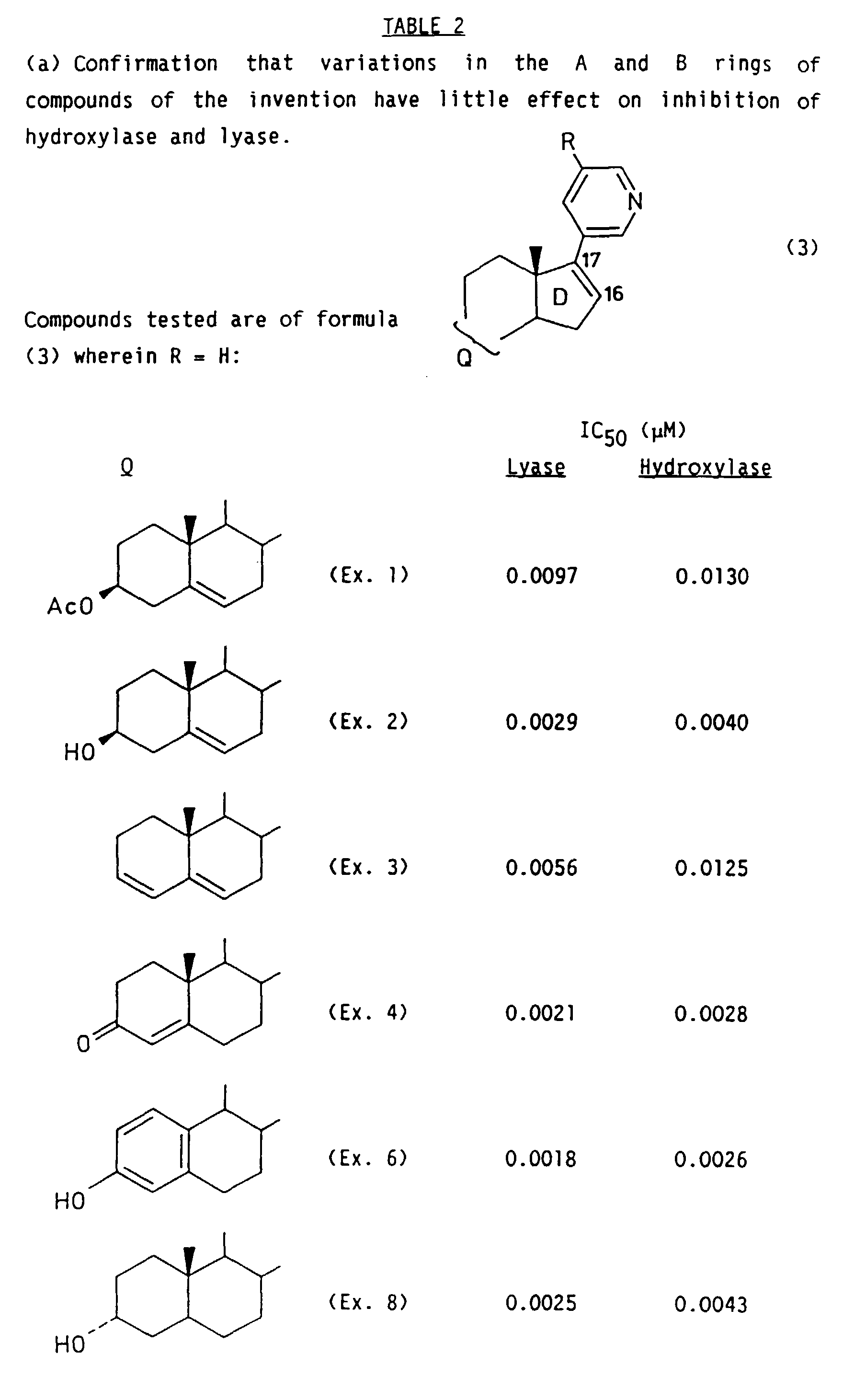

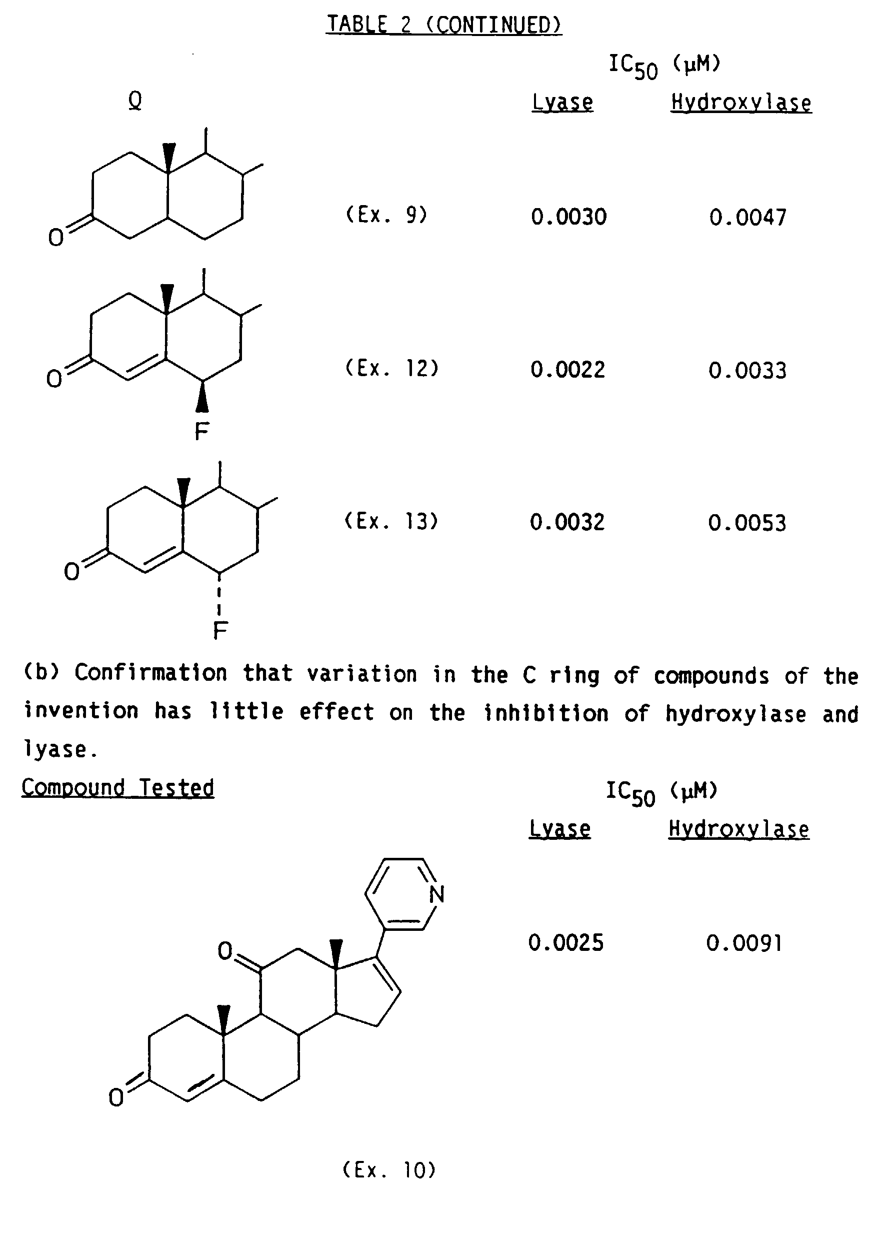

The comparative IC₅₀ figures for Ketoconazole are 0.026 against lyase and 0.065 against hydroxylase.

### Assay of aromatase activity

Aromatase activity was determined by the method of A. B. Foster et al., J. Med. Chem. 26, 50-54 (1983), using human placental microsomes. For the microsomes used, the Michaelis constant Kₘ for [1β - ³H] androstenedione was 0.039µM.

The compounds having a pregnenolone-like skeleton in the A and B rings, i.e. 3β-acetoxy-17-(3-pyridyl)androsta-5,16-diene and its 3-alcohol of Examples 1 and 2, had IC₅₀ > 20 µM. The compound having a progesterone-like skeleton in the A and B rings, i.e. 17-(3-pyridyl)-androsta-4,16-dien-3-one of Example 4 exhibited also aromatase inhibitory activity with IC₅₀ = 1µM.

### In vivo organ weight and endocrine test in mice

Male HWT mice, 12 weeks old, were treated daily for 2 weeks, with 5 animals per treatment group. The test compounds were the compound of Examples 1 and 4 (as representative of compounds of the invention having the pregenolone-like and progesterone-like skeletons respectively). Ketoconazole was also tested at three different doses. The test compounds were made up in 5% benzyl alcohol, 95% safflower oil, and were given i.p.. In addition to an untreated control group of animals, there was also a solvent control group which received the same volume of liquid as the test group (5ml/kg) but no test compound. All animals were sacrificed 24 hours after the last injection. Blood was collected by cardiac puncture into heparinized tubes, and the plasma used for RIA (radio immunoassay) of testosterone and luteinising hormone. The following organs were removed and weighed: adrenals, prostate, seminal vesicles, testes, kidneys. There was no significant body weight loss in any group of mice during the experiments.

Post mortem examination of the mice revealed oil/white deposits i.p. in those treated with compound of Ex. 1 and white deposits throughout the abdomen in those treated with compound of Ex. 4. In all these mice, all organs looked normal. In Ketoconazole-treated animals, adhesions were found in 2/5,2/5,4/5 of the low/middle/top dose groups. The gut and peritoneal wall seemed to be stuck to the seminal vesicles. The livers were brown in the middle/top dose groups.

The weights of organs found in the animals post mortem are shown in Table 3 below. The reductions in weight of all of the prostate, seminal vesicles, testes and kidneys were much greater for the test compounds of the invention than for Ketoconazole. Ketoconazole caused an increase in adrenal weight at the two highest doses, whereas the compounds of the invention had no significant effect, suggesting that they did not inhibit corticosterone biosynthesis.

**TABLE 3**

| Compound of Ex 1. | | | | | |
|---|---|---|---|---|---|
| Mean weight (mg.) ± standard error. | | | | | |
| Dose | Adrenals | Prostate | Seminal Vesicles | Testes | Kidneys |
| controls | 4.5 ± 0.1 | 10.1 ± 0.7 | 189 ± 9 | 146 ± 3 | 709 ± 17 |
| solvent controls | 4.5 ± 0.4 | 10.2 ± 1.3 | 171 ± 6 | 122 ± 7 | 615 ± 28 |
| 0.02mmol/ /kg/day | 4.3 ± 0.2 | 8.0 ± 0.6 | 136 ± 4 | 134 ± 4 | 604 ± 24 |
| 0.1 mmol /kg/day | 4.0 ± 0.2 | 5.3 ± 0.3 | 51 ± 6 | 95 ± 3 | 500 ± 8 |
| 0.5 mmol /kg/day | 4.7 ± 0.2 | 5.6 ± 0.6 | 25 ± 2 | 56 ± 2 | 449 ± 12 |

| Compound of Ex 4. | | | | | |
|---|---|---|---|---|---|
| Dose | Adrenals | Prostate | Seminal Vesicles | Testes | Kidneys |
| controls solvent | 4.3 ± 0.4 | 8.4 ± 0.2 | 165 ± 18 | 142 ± 8 | 652 ± 45 |
| controls | 4.4 ± 0.0 | 9.2 ± 0.9 | 152 ± 9 | 122 ± 8 | 589 ± 24 |
| 0.02mmol/kg/day | 4.7 ± 0.2 | 5.9 ± 0.8 | 108 ± 4 | 117 ± 9 | 599 ± 29 |
| 0.1 mmol/kg/day | 4.6 ± 0.4 | 6.4 ± 0.5 | 61 ± 9 | 105 ± 5 | 549 ± 28 |
| 0.5 mmol/kg/day | 4.9 ± 0.1 | 4.1 ± 0.5 | 25 ± 1 | 59 ± 2 | 468 ± 15 |

| Ketoconazole | | | | | |
|---|---|---|---|---|---|
| Dose | Adrenals | Prostate | Seminal Vesicles | Testes | Kidneys |
| controls solvent | 4.2 ± 0.2 | 8.9 ± 0.8 | 193 ± 8 | 145 ± 4 | 670 ± 12 |
| controls | 4.7 ± 0.4 | 9.3 ± 1.2 | 198 ± 18 | 146 ± 3 | 615 ± 25 |
| 0.01mmol/ /kg/day | 4.8 ± 0.2 | 9.1 ± 0.8 | 235 ± 18 | 141 ± 5 | 637 ± 22 |
| 0.225 mmol/kg/day | 6.1 ± 0.3 | 10.8 ± 1.4 | 171 ± 5 | 127 ± 7 | 574 ± 23 |
| 0.5 mmol/kg/day | 6.9 ± 0.3 | 9.3 ± 0.9 | 179 ± 20 | 133 ± 6 | 710 ± 30 |

The results indicate the inhibition by the components of the invention of androgen and particularly testosterone synthesis. They are confirmed by endocrinological results shown in Table 4. Although the solvent itself produced marked depression of testosterone levels, probably due to stress on the animals, the further decrease resulting from the administration of test compounds was much more marked for the compounds of the invention than for ketoconazole. The rise in LH levels is ascribed to a feedback mechanism associated with depletion of testosterone.

**TABLE 4**

| Endocrinological Results (Mean ± standard error) | | |
|---|---|---|
| | Testosterone nM | LH ng/ml |
| Compound of Ex. 1 | | |
| Control Solvent | 9.8 ± 5.6 | 0.63 ± 0.16 |
| Control | 2.5 ± 1.2 | 0.80 ± 0.09 |
| 0.02Mmol/Kg/Day | 2.7 ± 0.5 | 3.4 ± 0.5 |
| 0.1Mmol/Kg/Day | 0.2 ± 0.1 | 2.55 ± 0.45 |
| 0.5Mmol/Kg/Day | 0.1 ± 0.0 | 2.25 ± 0.67 |

| Compound of Ex. 4 | | |
|---|---|---|
| Control solvent | 27.8 ± 11.4 | Not |
| Control | 11.0 ± 5.6 | determined |
| 0.02Mmol/Kg/Day | 4.5 ± 0.3 | |
| 0.1Mmol/Kg/Day | 3.5 ± 1.0 | |
| 0.5Mmol/Kg/Day | 0.4 ± 0.1 | |

| Ketoconazole | | |
|---|---|---|
| Control Solvent | 17.3 ± 7.1 | 0.66 ± 0.05 |
| Control | 1.3 ± 0.4 | 0.25 ± 0.13 |
| 0.1Mmol/Kg/Day | 0.9 ± 0.2 | 0.39 ± 0.14 |
| 0.225Mmol/Kg/Day | 0.7 ± 0.1 | 0.75 ± 0.02 |
| 0.5Mmol/Kg/Day | 0.4 ± 0.1 | 0.76 ± 0.03 |

## Claims

1. Compounds of the general formula (1) 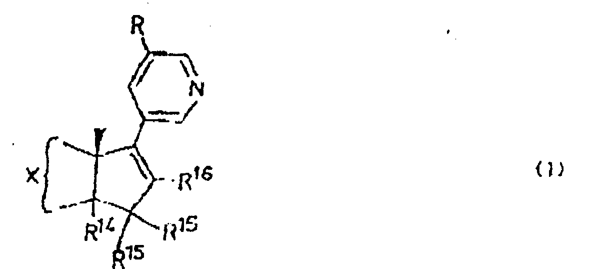 wherein X represents the residue of the A, B and C rings of a steroid, R represents a hydrogen atom or an alkyl group of 1-4 carbon atoms, R¹⁴ represents a hydrogen atom, a halogen atom or an alkyl group of 1 to 4 carbon atoms and each of the R¹⁵ substituents independently represents a hydrogen atom or an alkyl or alkoxy group of 1-4 carbon atoms, a hydroxy group or an alkylcarbonyloxy group of 2 to 5 carbon atoms or together represent an oxo or methylene group or R¹⁴ and one of the R¹⁵ groups together represent a double bond and the other R¹⁵ group represents a hydrogen atom or an alkyl group of 1 to 4 carbon atoms, and R¹⁶ represents a hydrogen atom, halogen atom, or an alkyl group of 1 to 4 carbon atoms, in the form of the free bases or pharmaceutically acceptable acid addition salts, with the proviso that 3β-acetoxy-17-(3-pyridyl)androsta-5,14,16-triene, 3β,15α- and 3β,15β-diacetoxy-17-(3-pyridyl)androsta-5,16-diene and 3β-methoxy-17-(3-pyridyl)-5α-androst-16-ene are claimed only for use in therapy.

2. Compounds according to Claim 1 wherein X represents the residue of
androstan-3α- or 3β-ol,
androst-5-en-3α- or 3β-ol,
androst-4-en-3-one,
androst-2-ene
androst-4-ene
androst-5-ene
androsta-5,7-dien-3α or 3β-ol,
androsta-1,4-dien-3-one
androsta-3,5-diene,
estra-1,3,5[10]-triene or
estra-1,3,5[10]-trien-3-ol,
each of which, where structurally permissible, can be further derivatised in one or more of the following ways:
- to form 3-esters
- to have one or more carbon to carbon ring double bonds in any of the 5,6-, 6,7-, 7,8-, 9,11- and 11,12-positions
- as 3-oximes
- as 3-methylenes
- as 3-carboxylates
- as 3-nitriles
- as 3-nitros
- as 3-desoxy derivatives
- to have one or more hydroxy, halo, C₁₋₄-alkyl, trifluoromethyl, C₁₋₄-alkoxy, C₁₋₄-alkanoyloxy, benzoyloxy, oxo, methylene or alkenyl substituents in the A, B or C-ring
- to be 19-nor.

3. Compounds according to Claim 1 or 2 which are saturated and unsubstituted at the 11- and 12- positions.

4. 17-(3-Pyridyl)androsta-5,16-dien-3β-ol,
17-(3-pyridyl)androsta-3,5,16-triene,
17-(3-pyridyl)androsta-4,16-dien-3-one,
17-(3-pyridyl)estra-1,3,5[10],16-tetraen-3-ol,
17-(3-pyridyl)-5α-androst-16-en-3α-ol
and their acid addition salts and 3-esters.

5. Compounds according to claim 1, 2 or 3 wherein R represents a hydrogen atom.

6. 17-(3-Pyridyl)-5α-androst-16-en-3-one,
17-(3-pyridyl)-androsta-4,16-diene-3,11-dione,
17-(3-pyridyl)-androsta-3,5,16-trien-3-ol,
6α-and 6β-fluoro-17-(3-pyridyl)androsta-4,16-dien-3-one
17-(3-pyridyl)androsta-4,16 dien-3,6-dione,
3α-trifluoromethyl-17-(3-pyridyl)androst-16-en-3β-ol
and their acid addition salts and 3-esters.

7. A pharmaceutical composition comprising a compound of the general formula (1) given in claim 1 or further defined by the feature of claim 2 or 3 or claimed in Claim 6 in association with a pharmaceutically acceptable carrier or diluent.

8. Compounds according to Claim 1, 2, 3 or 6, for use in the therapy of androgen-dependent disorders.

9. Compounds according to Claim 8 for use in treating prostatic cancer.

10. Compounds according to Claim 1, 2, 3 or 6, for use in the therapy of oestrogen-dependent disorders.

11. Compounds according to Claim 10 for use in treating breast cancer.

12. A pharmaceutical composition comprising a compound of the general formula given in claim 1 wherein R represents a hydrogen atom or a compound claimed in Claim 4, in association with a pharmaceutically acceptable carrier or diluent.

13. Compounds according to Claim 4 or 5, for use in the therapy of androgen-dependent disorders.

14. Compounds according to Claim 13 for use in treating prostatic cancer.

15. Compounds according to Claim 4 or 5, for use in the therapy of oestrogen-dependent disorders.

16. Compounds according to Claim 15 for use in treating breast cancer.

## Patentansprüche

1. Verbindungen der allgemeinen Formel (1) 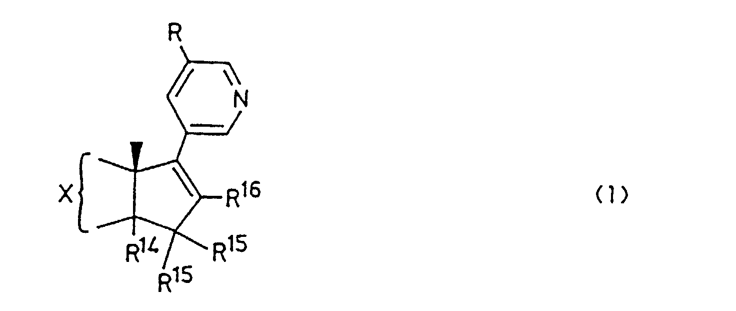 in der X für den Rest der A-, B- und C-Ringe eines Steroids steht, R für ein Wasserstoffatom oder eine Alkylgruppe mit 1-4 Kohlenstoffatomen steht, R¹⁴ für ein Wasserstoffatom, ein Halogenatom oder eine Alkylgruppe mit 1 bis 4 Kohlenstoffatomen steht und jeder der R¹⁵-Substituenten unabhängig für ein Wasserstoffatom oder eine Alkyl- oder Alkoxygruppe mit 1-4 Kohlenstoffatomen, eine Hydroxygruppe oder eine Alkylcarbonyloxygruppe mit 2 bis 5 Kohlenstoffatomen steht oder zusammen für eine Oxo- oder Methylengruppe stehen oder R¹⁴ und eine der R¹⁵-Gruppen zusammen für eine Doppelbindung stehen und die andere R¹⁵-Gruppe für ein Wasserstoffatom oder eine Alkylgruppe mit 1 bis 4 Kohlenstoffatomen steht und R¹⁶ für ein Wasserstoffatom, ein Halogenatom oder eine Alkylgruppe mit 1 bis 4 Kohlenstoffatomen steht, in Form der freien Basen oder pharmazeutisch annehmbarer Säureadditionssalze mit der Maßgabe, daß 3β-Acetoxy-17-(3-pyridyl)androsta-5,14,16-trien, 3β,15α- und 3β,15β-Diacetoxy-17-(3-pyridyl)androsta-5,16-dien und 3β-Methoxy-17-(3-pyridyl)-5α-androst-16-en nur für eine Verwendung in der Therapie beansprucht werden.

2. Verbindungen nach Anspruch 1, in denen X für den Rest
Androstan-3α- oder -3β-ol,
Androst-5-en-3α- oder -3β-ol,
Androst-4-en-3-on,
Androst-2-en,
Androst-4-en,
Androst-5-en,
Androsta-5,7-dien-3α- oder 3β-ol,
Androsta-1,4-dien-3-on,
Androsta-3,5-dien,
Estra-1,3,5[10]-trien oder
Estra-1,3,5[l0]-trien-3-ol,
steht, von denen jeder, wo strukturell zulässig, weiter in einer oder mehreren der folgenden Weisen derivatisiert sein kann:
- daß 3-Ester gebildet werden,
- daß eine oder mehrere Kohlenstoff-Kohlenstoff-Ring-Doppelbindungen in einer beliebigen der 5,6-, 6,7-, 7,8-, 9,11- und 11,12-Positionen vorliegen,
- als 3-Oxime,
- als 3-Methylene,
- als 3-Carboxylate,
- als 3-Nitrile,
- als 3-Nitrogruppen,
- als 3-Desoxyderivate,
- daß ein oder mehrere Hydroxy-, Halogen-, C₁₋₄-Alkyl-, Trifluormethyl-, C₁₋₄-Alkoxy-, C₁₋₄-Alkanoyloxy-, Benzoyloxy-, Oxo-, Methylen- oder Alkenylsubstituenten in dem A-, B- oder C-Ring vorliegen,
- daß er 19-nor ist.

3. Verbindungen nach Anspruch 1 oder 2, die gesättigt und an den Positionen 11 und 12 unsubstituiert sind.

4. 17-(3-Pyridyl)androsta-5,16-dien-3β-ol,
17-(3-Pyridyl)androsta-3,5,16-trien,
17-(3-Pyridyl)androsta-4,16-dien-3-on,
17-(3-Pyridyl)estra-1,3,5[10],16-tetraen-3-ol,
17-(3-Pyridyl)-5α-androst-16-en-3α-ol
und ihre Säureadditionssalze und 3-Ester.

5. Verbindungen nach Anspruch 1, 2 oder 3, wobei R für ein Wasserstoffatom steht.

6. 17-(3-Pyridyl)-5α-androsta-16-en-3-on,
17-(3-Pyridyl)-androsta-4,16-dien-3,11-dion,
17-(3-Pyridyl)-androsta-3,5,16-trien-3-ol,
6α- und 6β-Fluor-17-(3-pyridyl)androsta-4,16-dien-3-on,
17-(3-Pyridyl)androsta-4,16-dien-3,6-dion,
3α-Trifluormethyl-17-(3-pyridyl)androst-16-en-3β-ol
und ihre Säureadditionssalze und 3-Ester.

7. Pharmazeutische Zusammensetzung, umfassend eine Verbindung der allgemeinen Formel (1), die in Anspruch 1 angegeben ist oder weiter durch das Merkmal von Anspruch 2 oder 3 definiert ist oder die in Anspruch 6 beansprucht wird, in Verbindung mit einem pharmazeutisch annehmbaren Träger oder Verdünnungsmittel.

8. Verbindungen nach Anspruch 1, 2, 3 oder 6 für eine Verwendung bei der Therapie von Androgen-abhängigen Erkrankungen.

9. Verbindungen nach Anspruch 8 für eine Verwendung bei der Behandlung von Prostatakrebs.

10. Verbindungen nach Anspruch 1, 2, 3 oder 6 für eine Verwendung bei der Behandlung von Östrogen-abhängigen Erkrankungen.

11. Verbindungen nach Anspruch 10 für eine Verwendung bei der Behandlung von Brustkrebs.

12. Pharmazeutische Zusammensetzung, umfassend eine Verbindung der in Anspruch 1 angegebenen allgemeinen Formel, in der R für ein Wasserstoffatom steht, oder eine in Anspruch 4 beanspruchte Verbindung in Verbindung mit einem pharmazeutisch annehmbaren Träger oder Verdünnungsmittel.

13. Verbindungen nach Anspruch 4 oder 5 für eine Verwendung bei der Therapie von Androgen-abhängigen Erkrankungen.

14. Verbindungen nach Anspruch 13 für eine Verwendung bei der Behandlung von Prostatakrebs.

15. Verbindungen nach Anspruch 4 oder 5 für eine Verwendung bei der Therapie von Östrogen-abhängigen Erkrankungen.

16. Verbindungen nach Anspruch 15 zur Verwendung bei der Behandlung von Brustkrebs.

## Revendications

1. Composés de formule générale (1) : 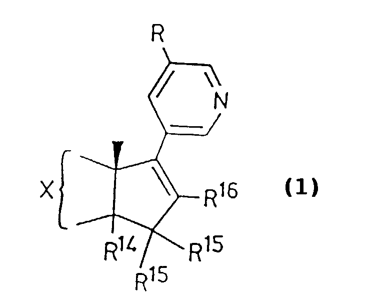 dans laquelle X représente le résidu des cycles A, B et C d'un stéroïde, R représente un atome d'hydrogène ou un groupe alkyle de 1 à 4 atomes de carbone, R¹⁴ représente un atome d'hydrogène, un atome d'halogène ou un groupe alkyle de 1 à 4 atomes de carbone et chacun des substituants R¹⁵ représente indépendamment un atome d'hydrogène ou un groupe alkyle ou alcoxy de 1 à 4 atomes de carbone, un groupe hydroxy ou un groupe alkylcarbonyloxy de 2 à 5 atomes de carbone ou représentent ensemble un groupe oxo ou méthylène ou R¹⁴ et l'un des groupes R¹⁵ représentent ensemble une double liaison et l'autre groupe R¹⁵ représente un atome d'hydrogène ou un groupe alkyle de 1 à 4 atomes de carbone, et R¹⁶ représente un atome d'hydrogène, un atome d'halogène, ou un groupe alkyle de 1 à 4 atomes de carbone, sous la forme de leurs bases libres ou de sels d'addition avec un acide pharmaceutiquement acceptables, sous réserve que le 3β-acétoxy-17-(3-pyridyl)androsta-5,14,16-triène, le 3β-15α-et le 3β-15β-diacétoxy-17-(3-pyridyl)androsta-5,16-diène et le 3β-méthoxy-17-(3-pyridyl)-5α-androst-16-ène sont seulement revendiqués pour usage en thérapie.

2. Composés selon la revendication 1, dans lesquels X représente le résidu de
l'androstan-3α- ou 3β-ol,
l'androst-5-èn-3α- ou 3β-ol,
l'androst-4-èn-3-one,
l'androst-2-ène,
l'androst-4-ène,
l'androst-5-ène,
l'androsta-5,7-dièn-3α- ou 3β-ol,
l'androsta-1,4-dièn-3-one,
l'androsta-3,5-diène,
l'oestra-1,3,5[10]-triène ou
l'oestra-1,3,5[10]-trièn-3-ol,
chacun d'eux, si leur structure le permet, peut être en outre modifié selon l'une ou plusieurs des modes suivants :
- pour former des 3-esters
- pour présenter une ou plusieurs doubles liaisons cycliques carbone carbone dans l'une quelconque des positions 5,6-, 6,7-, 7,8-, 9,11- et 11,12-
- sous forme de dérivés 3-oximes
- 3-méthylènes,
- 3-carboxylates
- 3-nitriles
- 3-nitros
- 3-désoxy
- pour présenter un ou plusieurs substituants hydroxy, halogéno, alkyle en C₁ à C₄, trifluorométhyle, alcoxy en C₁ à C₄, alcanoyloxy en C₁ à C₄, benzoyloxy, oxo, méthylène ou alcényle sur le cycle A, B ou C
- pour être 19-nor.

3. Composés selon la revendication 1 ou 2 qui sont saturés et non substitués en positions 11 et 12.

4. Le 17-(3-pyridyl)androsta-5,16-dièn-3β-ol,
le 17-(3-pyridyl)androsta-3,5,16-triène,
la 17-(3-pyridyl)androsta-4,16-dièn-3-one,
le 17-(3-pyridyl)oestra-1,3,5[10],16-tétraèn-3-ol,
le 17-(3-pyridyl)-5α-androst-16-èn-3α-ol,
et leurs sels d'addition et 3-esters.

5. Composés selon la revendication 1, 2 ou 3 dans lesquels R représente un atome d'hydrogène.

6. La 17-(3-pyridyl)-5α-androst-16-èn-3-one,
la 17-(3-pyridyl)-5α-androsta-4,16-dièn-3,11-dione,
le 17-(3-pyridyl)-androsta-3,5,16-trièn-3-ol,
la 6α- et 6β-fluoro-17-(3-pyridyl)androsta-4,16-dièn-3-one,
la 17-(3-pyridyl)androsta-4,16-dièn-3,6-dione,
la 3α-trifluorométhyl-17-(3-pyridyl)androst-16-én-3β-ol
et leurs sels d'addition d'un acide et les 3-esters.

7. Composition pharmaceutique comprenant un composé de formule générale (1) donnée dans la revendication 1 ou définie en outre par les caractéristiques de la revendication 2 ou 3 ou revendiqué à la revendication 6 en association avec un véhicule ou un diluant pharmaceuti-quement acceptable.

8. Composés selon la revendication 1, 2, 3 ou 6, destinés à la thérapie des troubles dépendants des hormones androgènes.

9. Composés selon la revendication 8 destinés au traitement du cancer de la prostate.

10. Composés selon la revendication 1, 2, 3 ou 6, destinés à la thérapie des troubles dépendants des hormones oestrogènes.

11. Composés selon la revendication 10 destinés au traitement du cancer du sein.

12. Composition pharmaceutique comprenant un composé de la formule générale donnée à la revendication 1 dans laquelle R représente un atome d'hydrogène ou un composé revendiqué à la revendication 4, en association avec un véhicule ou un diluant pharmaceutiquement acceptable.

13. Composés selon la revendication 4 ou 5 destinés à la thérapie des troubles dépendants des hormones androgènes.

14. Composés selon la revendication 13 destinés au traitement du cancer de la prostate.

15. Composés selon la revendication 4 ou 5, destinés à la thérapie des troubles dépendants des hormones oestrogènes.

16. Composés selon la revendication 15 destinés au traitement du cancer du sein.
